# EUROPEAN PATENT APPLICATION

(11) **EP 2 604 607 A1**
(43) Date of publication of application: **19.06.2013**
(21) Application number: 11816484.7
(22) Date of filing: 11.08.2011
(51) Int. Cl.: C07D 471/04, A61K 31/437, A61P 1/16, A61P 29/00

(54) **PROPHYLACTIC OR THERAPEUTIC AGENT FOR NON-ALCOHOLIC STEATOHEPATITIS**

(30) Priority: 12.08.2010 JP 2010180656
(71) Applicant: Kyorin Pharmaceutical Co., Ltd., Tokyo 101-8311 (JP)
(72) Inventor: MATSUI, Toshiyuki, Shimotsuga-gun Tochigi 329-0114 (JP); IDE, Tomohiro, Shimotsuga-gun Tochigi 329-0114 (JP); TSUNODA, Masaki, Shimotsuga-gun Tochigi 329-0114 (JP); OGATA, Tomomi, Shimotsuga-gun Tochigi 329-0114 (JP); ITO, Minoru, Shimotsugu-gun Tochigi 329-0114 (JP)
(74) Representative: Vossius & Partner
(86) International application number: PCT/JP2011/068344
(87) International publication number: WO 2012/020821

(57) **Abstract**

An object of the present invention is to provide an agent for prevention or treatment of a fatty liver disease, preferably NAFLD, more preferably NASH. The present invention provides an agent for prevention or treatment of a fatty liver disease containing ibudilast as an active agent.

## Description

### TECHNICAL FIELD

The present invention relates to an agent for prevention or treatment of nonalcoholic fatty liver disease, in particular nonalcoholic steatohepatitis, comprising ibudilast as an active ingredient.

### BACKGROUND ART

All fatty liver diseases that include fatty liver similar to alcoholic liver disease which occurs in non-drinkers are called nonalcoholic fatty liver disease (NAFLD). The induction of the fatty acid synthesis in the liver of NAFLD patients is constantly increased. The fatty acid synthesis in the liver is considered to be an important factor involved in the fatty liver formation which causes metabolic syndrome (Non-Patent Document 1).

Furthermore, hepatic overexpression of SREBP-1, a key transcription factor that regulates hepatic fatty acid synthesis, develops fatty liver in mice (Non-Patent Document 2). Conversely, it is known that knockout of SREBP-1 attenuated fatty liver in mice (Non-Patent Document 3).

NAFLD is generally classified roughly as simple fatty liver with a favorable prognosis and nonalcoholic steatohepatitis (NASH) which accompanies inflammation and fibrosis correlate with poor prognosis, and NASH is regarded as a severe type of NAFLD (Non-Patent-Documents 4 to 7).

The proposed mechanism for pathogenesis and progression of NASH involves the two-hit theory currently (Non-Patent Document 4). In two hit theory, NASH develops and progresses by simple fatty liver which develops by environmental and genetic factors as the "first hit", added to oxidative stress and inflammatory cytokine induced by fatty liver as the "second hit" (Non-Paten Documents 8 and 9).

Therapy in metabolic syndrome as pathologic basis of NASH is important. Therefore, insulin-sensitizing agents, antioxidants, lipid-lowering agents, hepatoprotective agents and angiotensin II receptor blockers are used (Non-Patent Document 4).

However, it is recognized that there is no medicine which has recommendable evidence in therapeutic strategies for NASH to date. For example, an insulin-sensitizing agent, pioglitazone, was expected as a treating agent for NASH. However, according to the results of phase III (PIVENS), pioglitazone has no improvement on fibrosis and cannot meet original criteria (Non-Patent Document 10). Moreover, there are concerns of side effect by pioglitazone, such as fracture risk, body weight gain, and onset or worsening of cardiac failure (Non-Patent Document 11).

On the other hand, ibudilast is widely used in clinical as a cerebrovascular disorder improving agent, a treating agent for bronchial asthma and a treating agent for allergic conjunctivitis and the safety thereof has been verified. As the action of ibudilast, various actions, such as enhancement of cerebrovascular relaxant of prostacyclin (PGI₂) (Non-Patent Document 12), enhancement of platelet aggregation inhibition action (Non-Patent-Document 13), inhibitory effects on airway contraction, leukotriene antagonistic action, leukotriene release inhibitory action (Non-Patent-Document 14), PDE inhibitory action (Patent-Document 1) and inhibitory action of activation of migration inhibitory factor (MIF) (Non-Patent Document 15) are known. However, effects on fatty liver disease, NAFLD, NASH and fatty liver have never known.

### CITATION LIST

### PATENT DOCUMENT

[Patent Document 1] WO 2004/050091 1

### NON-PATENT DOCUMENTS

[Non-Patent Document 1] Shinji, T. et al., 2005, J. Clin. Invest., 115:1139-1142
[Non-Patent Document 2] Shimano, H., et al., 1996, J Clin. Invest., 98:1575-1584
[Non-Patent Document 3] Yahagi, N. et al., 2002, J Biol. Chem., 277:19353-19357
[Non-Patent Document 4] Toshiji Saibara et al., NASH Shinryo best approach (the best approach to the treatment of NASH), Chugai-igakusha, December 5, 2008
[Non-Patent Document 5] Schaffner, F., et al., 1986, Prog. Liver., Dis. 8:283-298
[Non-Patent Document 6] Neuschwander-Tetri, B. A., et al., 2003. Hepatology , 37:1202-1219
[Non-Patent Document 7] Erickson, S.K., et al. 2009, J. Lipid. Res., 50:S412-416
[Non-Patent Document 8] Day, C. P., et al. 1998, Gastroenterology., 114:842-845
[Non-Patent Document 9] Browning, J.D., et al. 2004, J. Clin. Invest. 114:147-152
[Non-Patent Document 10] Sanyal, A. J., et al. 2010, N. Engl. J. Med., 362:1675-1685
[Non-Patent Document 11] Harrison, S.A., et al. 2010, Hepatology, 51:366-369
[Non-Patent Document 12] Ohashi, M., et al. 1986, Arch. Int. Pharmacodyn. Ther., 280:216-229
[Non-Patent Document 13] Ohashi, M., et al. 1986, Arch. Int. Pharmacodyn. Ther., 283:321-334
[Non-Patent Document 14] Mitsuo Ohashi, 1989, Zensoku (asthma), 2:103-107
[Non-Patent Document 15] Cho, Y, et al. 2010, Proc. Natl. Acad. Sci. USA., 107:11313-11318

### SUMMARY OF THE INVENTION

### PROBLEMS TO BE SOLVED BY THE INVENTION

An object of the present invention is to provide an agent for prevention or treatment of fatty liver disease, which is effective for NAFLD, especially NASH.

### MEANS FOR SOLVING THE PROBLEMS

The inventors have discovered that ibudilast improves all of fatty liver, hepatic inflammation and hepatic fibrosis which are main pathological conditions of NASH, and accomplished the invention.

Namely, the present invention relates to the following (1) to (4).
(1) An agent for preventing or treating a fatty liver disease comprising ibudilast as an active ingredient;
(2) The agent described in (1), in which the fatty liver disease is nonalcoholic fatty liver disease;
(3) The agent described in (2), in which the nonalcoholic fatty liver disease is nonalcoholic steatohepatitis; and
(4) The agent described in (2), in which the nonalcoholic fatty liver disease is simple fatty liver.

### EFFECT OF THE INVENTION

Since the agent for prevention or treatment of fatty liver disease of the present invention comprises ibudilast, it can inhibit all of fatty acid synthesis in a liver, fatty liver, hepatic inflammation and hepatic fibrosis, and therefore can effectively prevent or treat NAFLD, especially NASH.

### BRIEF DESCRIPTION OF THE DRAWINGS

[Fig. 1] Fig. 1 shows the effect of ibudilast on the hepatic fatty acid synthesis (10 and 100 mg/kg, administration of twice a day). The numerical value is the mean value ± the standard error (n=5 to 6, ^{*}p<0.05 vs. vehicle).
[Fig. 2] Fig. 2 shows the effect of ibudilast on hepatic triglyceride content (10 and 100 mg/kg, administration of twice a day). The numerical value is the mean value ± the standard error (n=5 to 6, ^{*}p<0.05 vs. vehicle).
[Fig. 3] Fig. 3 shows the effect of ibudilast on hepatic triglyceride content (10, 30 and 100 mg/kg, administration of twice a day). The numerical value is the mean value ± the standard error (n=6, ^{*}p<0.05 vs. vehicle).
[Fig. 4] Fig. 4 shows the effect of ibudilast on TNFα mRNA expression in the liver (10, 30 and 100 mg/kg, administration of twice a day). The numerical value is the mean value ± the standard error (n=9 to 10, ^{*}p<0.05 vs. vehicle).
[Fig. 5] Fig. 5 shows the effect of ibudilast on MCP-1 mRNA expression in the liver (10, 30 and 100 mg/kg, administration of twice a day). The numerical value is the mean value ± the standard error (n=9 to 10, ^{*}p<0.05 vs. vehicle).
[Fig. 6] Fig. 6 shows the effect of ibudilast on IL-1β mRNA expression in the liver (10, 30 and 100 mg/kg, administration of twice a day). The numerical value is the mean value ± the standard error (n=9 to 10, ^{*}p<0.05 vs. vehicle).
[Fig. 7] Fig. 7 shows the effect of ibudilast on TGFβ mRNA expression in the liver (10, 30 and 100 mg/kg, administration of twice a day). The numerical value is the mean value ± the standard error (n=9 to 10, ^{*}p<0.05 vs. vehicle).
[Fig. 8] Fig. 8 shows the effect of ibudilast on collal mRNA expression in the liver (10, 30 and 100 mg/kg, administration of twice a day). The numerical value is the mean value ± the standard error (n=9 to 10, ^{*}p<0.05 vs. vehicle).

### EMBODIMENTS FOR CARRYING OUT THE INVENTION

Hereinafter, the present invention will be described in detail.

The agent for prevention or treatment of fatty liver disease in the present invention comprises ibudilast as an active ingredient.

Ibudilast is a known compound represented by the following formula (1), and can be prepared according to a known preparation method (such as JP-B-52-29318).

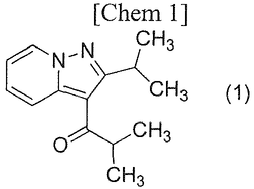

In the present invention, the "fatty liver disease" means a general name for diseases in which neutral fat is deposited in the hepatocyte and causes hepatopathy. It includes alcoholic liver disease and nonalcoholic fatty liver disease (NAFLD).

In the present invention, the "nonalcoholic fatty liver disease" has the same definitions as nonalcoholic fatty liver and nonalcoholic hepatic steatosis. Examples of the nonalcoholic fatty liver disease include nonalcoholic steatohepatitis (NASH) and simple fatty liver.

For example, the nonalcoholic fatty liver disease (NAFLD) is a hepatopathy which is characterized by mainly macrovesicular fatty deposit in liver and is similar to the findings in the liver tissue of alcoholic hepatopathy despite lack of the significant history of alcohol drinking, and it is defined as a concept of the disease including simple fatty liver with a good prognosis and progressive NASH *["*NASH · NAFLD no shinryo gaido" (Diagnostic Guide of NASH and NAFLD) (edited by The Japan Society of Hepatology, BUNKODO, August 2006)].

In addition, examples of features of nonalcoholic fatty liver disease (NAFLD) include the following features.
1. There is no significant history of alcohol drinking (amount of alcohol: 20g or less/day).
2. There is no chronic liver disease which has clear cause, such as viral (HCV, HBV) or autoimmune.
3. Metabolic syndrome, obesity, diabetes, hyperlipidemia, hypertension, hyperuricemia, sleep apnea syndrome or the like, are risk factors. The possibility of simple fatty liver<NASH increases in persons having multiple risk factors.
4. The cause also includes various diseases and medicines which lead to abnormal lipid metabolism or abnormal mitochondrial function.

Nonalcoholic steatohepatitis (NASH) is defined, for example, as follows *("*Diagnosis guide for NASH and NAFLD" (edited by The Japan Society of Hepatology, BUNKODO, August 2006)).
1. Matteoni (Matteoni, C. A., et al. 1999. Gastroenterology. 116: 1413-1419) classified NAFLD, into four types. Type 1: simple fatty liver, Type 2: steatohepatitis, Type 3: fatty liver necrosis (accompanied by ballooning degeneration.), Type 4: hepatocyte necrosis accompanied by mallory bodies or fibrosis (accompanied by ballooning degeneration). Type 3 and type 4 which has a significantly high frequency in progress to cirrhosis and the death related to the liver are defined as nonalcoholic steatohepatitis (NASH) from the examination of a long-term prognosis.
2. American Association for the Study of Liver Diseases defined examples of findings essential for NASH include fatty metamorphosis (macrovesicular>microvesicular, mainly present in the centrilobular portion), intralobular inflammation (mild, migration of neutrophil and monocyte), ballooning degeneration of hepatocyte (mainly present in the surrounding of fatty change and centrilobular portion) at the Single Topic Conference 2002 (Neuschwander-Tetri, B. A., et al. 2003. Hepatology, 37: 1202-1219), and defined type 3 and type 4 (classification by Matteoni) of NAFLD as NASH.
3. Brunt (Brunt, E. M., et al. 1999. Am. J. Gastroenterol. 94: 2467-2474) classified the progress of NASH into four stages according to the level of fibrosis: stage 1: centrilobular portion (Zone 3), stage 2: stage 1+portal region, stage 3: formation of cross-linking, stage 4: cirrhosis.

In the present invention, the simple fatty liver is a case of fatty liver diseases which is characterized by only fatty deposits in hepatocytes and is not accompanied by necrosis of hepatocytes, inflammation and fibrosis.

Examples of the fatty liver disease to which the agent for prevention and treatment in the present invention is applied include preferably the nonalcoholic fatty liver disease NAFLD), more preferably the nonalcoholic steatohepatitis (NASH) or the simple fatty liver, and especially preferably the nonalcoholic steatohepatitis.

As described above, the two hit theory is recognized as a mechanism behind the onset and progress of NASH (Toshiji Saibara et al., NASH Shinryo best approach (the best approach to the treatment of NASH), Chugai-igakusha, December 5, 2008).

In two hit theory, NASH develops and progresses through the following mechanism behind the onset and progression. Firstly, accumulation of fat in liver (fatty liver) occurs as the first hit. Then, NASH occurs and progresses by onset of hepatic inflammation and hepatic fibrosis due to oxidative stress, inflammatory cytokine or the like caused by the fatty liver as the second hit. The onset and progression of NASH lead to cirrhosis and hepatoma.

Regarding the first hit, ibudilast firstly improves fatty liver by inhibiting accumulation of fat in a liver through inhibition of the lipogenic activity in liver and decrease in the hepatic triglyceride content. Regarding the second hit, ibudilast improves hepatic inflammation and hepatic fibrosis by inhibiting lipid droplet and migration of inflammatory cells in the liver, inhibiting oxidative stress and expression level of inflammatory cytokines and inhibiting expression of a gene relating to hepatic inflammation and hepatic fibrosis. Based on the above mechanism, it is considered that ibudilast can effectively inhibit a mechanism of the onset and progression of NASH.

The agent for prevention or treatment of fatty liver disease in the present invention comprises ibudilast and, if necessary, can be mixed with well-known pharmaceutically acceptable carriers. The carrier which can be optionally mixed may change depending on the dosage form, the administration form, or the like. Examples of the carrier include excipients, bonding agents, disintegrants, lubricants, corrigent, flavors, colorants and sweeteners, and the like.

Moreover, the agent for prevention or treatment of fatty liver disease in the present invention can be used in various pharmaceutically acceptable forms. Preferable examples of the form include capsules, powders, tablets, granules, pellets, injections, liquid medicines, ointments and patches, and the like.

Therefore, the agent for prevention or treatment of fatty liver disease in the present invention can be administered to the patient in a form for oral administration and parenteral administration. Especially, among these, oral preparation is preferable in consideration of ease of use for a patient.

The amount of ibudilast in the agent for prevention or treatment of fatty liver disease in the present invention can be appropriately changed according to patient's age, weight, symptom and route of administration or the like.

In the oral administration for adults (about 60 kg), ibudilast is preferably administered at a dose of 10 mg to 200 mg per once, more preferably 10 mg to 60 mg per once and twice to three times a day.

Furthermore, in the injection form for adults (about 60 kg), ibudilast is preferably administered at a dose of 10 mg to 200 mg per once, more preferably, 10 mg to 60 mg per once and twice to three times a day.

### EXAMPLES

Next, the present invention will be described by specific examples. However, the invention is not limited to these examples.

### Example 1

### Inhibitory effects of ibudilast on Fatty Liver (Evaluations using fatty liver model)

In NAFLD, it is regarded that the increase of the fatty acid synthesis in the liver is an important factor involved in the fatty liver formation (Shinji, T., et al. 2005. J. Clin.Invest. 115 : 1139-1142). Therefore, with reference to methods described in Delzenne *et al.* (Delzenne, N. M., et al., 1997, J Hepatol., 26: 880-885) and Tsuchida *et al.* (Tsuchida. A., et al., 2004, J. Bio. Chem., 279: 30817-30822), effects of ibudilast on the fatty liver were examined by using re-fed mice in which the fatty acid synthesis in the liver is enhanced.

After 48 hours of fasting, male C57BI/6J mice at 8 weeks of age (CLEA Japan, Inc.) were refed for four hours. To the vehicle group as a control, 2% (v/v) of PEG-60 hydrogenated castor oil (NIKKOL HCO-60, Nikko Chemicals Co., Ltd.) was orally administrated before fasting and after fasting periods of 12, 24, 36, and 48 hours. To ibudilast groups, 10 or 100 mg/kg of ibudilast which was dissolved to 2% (v/v) of PEG-60 hydrogenated castor oil was orally administered before fasting and after fasting periods of 12, 24, 36, and 48 hours.

The mice were refed with standard diet (CE-2, CLEA Japan, Inc.) after 30 minutes of the oral administration after fasting periods of 48 hours, and the mice were anatomized after refeeding periods of four hours. The content of triglyceride and biosynthesis of fatty acid in isolated liver were measured.

Triglyceride was extracted from the liver by using improved method of Folch *et al.* (Folch, J., et al. 1957. J. Biol. Chem., 226: 497-509). Tissues were homogenized by using chloroform-methanol mixture 2:1 (v/v).

The obtained homogenate was shaken for one hour to extract a lipid fraction, followed by obtaining the supernatant by centrifugation to dry. The extraction was carried out twice in the same procedure from sediment of the tissues and the obtained supernatant was mixed together to dry over. The dried extract was dissolved to 4% (v/v) of Triton X-100 and the triglyceride concentration was measured using Liquitech TG-II reagent (Roche Diagnostics K. K.). The result was shown by the mean value ± the standard error. The statistical analysis was performed using Williams' multiple comparison test where the level of statistical significance was less than 5%.

The fatty acid synthesis in the liver was measured using the fatty acid synthesis from the acetic acid. The tissue was incubated in 95% (v/v) O₂, 5% (v/v) CO₂, 0.5 mM acetic acid (0.25 µCi/mL, [1-¹⁴C]acetic acid) and Krebs-Ringer phosphate HEPES buffer (pH 7.4) containing 0.2% (v/v) BSA for two hours at 37°C. Then, to the isolated tissue, the ethanol solution containing 15% (w/v) of potassium hydroxide was added, and incubated for two hours at 85°C to saponify.

After adding petroleum ether and then shaking for 30 minutes, the ether layer as the upper layer obtained by centrifugation was removed. After the same procedure was carried out twice, the aqueous layer was adjusted using hydrochloric acid to pH1. After adding petroleum ether and shaking for 30 minutes, the ether layer as the upper layer obtained by centrifugation was collected. The same procedure was carried out twice. After mixing the collected ether layer together, obtained solution was dried over. After the dried extract was dissolved to chloroform, purified water was added thereto followed by shaking for 30 minutes.

After centrifugation, the chloroform layer was obtained as the lower layer. After the dried residue was dissolved to methanol, the scintillation cocktail was mixed with the obtained solution to measure radioactivity using liquid scintillation counter (Tri-Crab 1900CA, PerkinElmer). The result was shown as the mean value ± the standard error. The statistical analysis was performed using Williams' multiple comparison test where the level of statistical significance was less than 5%.

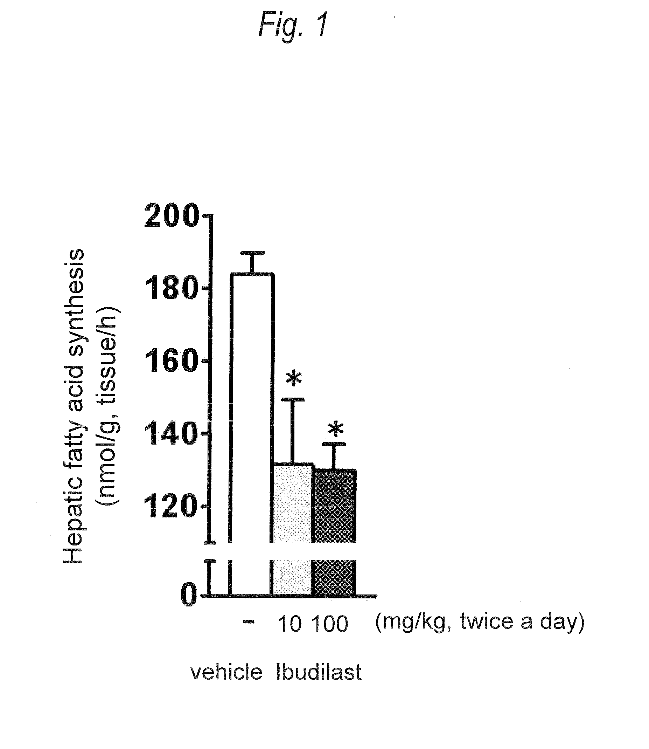

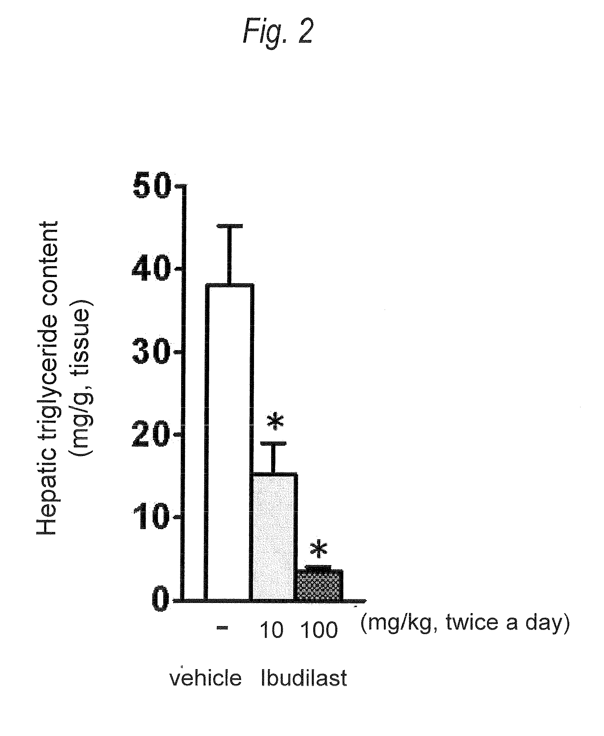

Ibudilast decreased the fatty acid synthesis in the liver which was regarded to be important to form a fatty liver in NAFLD (Fig. 1). Furthermore, the content of triglyceride in the liver which is an index of the fatty liver was also significantly decreased (Fig. 2). Accordingly, it was shown that ibudilast was excellent inhibitory effects on the fatty acid synthesis in the liver and on the development of fatty liver.

### Example 2

### Inhibitory effect of ibudilast on Fatty Liver (Evaluation using obesity-related fatty liver model)

Obesity and insulin resistance are regarded as a major pathologic basis in NAFLD (Kristina, M., et al. 2006, J Clin Endocrinol Metab., 91: 4753-4761). Therefore, effect of ibudilast on fatty liver was evaluated using B6.V-Lep^{ob}/J mice (ob/ob; Charles River Laboratories Japan, Inc.), a model of obesity-related insulin resistance and fatty liver.

Male ob/ob mice (Charles River Laboratories Japan, Inc.) at seven weeks of age were used. To the vehicle group as a control, 2% (v/v) of PEG-60 hydrogenated castor oil (NIKKOL HCO-60, Nikko Chemicals Co., Ltd.) was orally administrated twice a day. To ibudilast groups, 10, 30 or 100 mg/kg of ibudilast which was dissolved to 2% (v/v) of PEG-60 hydrogenated castor oil was orally administered twice a day.

At 15 to 21 hours after the end of two weeks administration period, the mice were dissected in the fed state. The content of triglyceride in isolated liver was measured.

Triglyceride was extracted from the liver by using improved method of Folch *et al.* (Folch, J., et al. 1957. J. Biol. Chem., 226: 497-509). Tissues were homogenized by using chloroform methanol mixture 2:1 (v/v).

The obtained homogenate was shaken for one hour to extract a lipid fraction, followed by obtaining the supernatant by centrifugation to dry. The extraction was carried out twice in the same procedure from sediment of the tissues and the obtained supernatant was mixed together to dry over. The dried extract was dissolved to 4% (v/v) of Triton X-100 and the triglyceride concentration was measured using Liquitech TG-II reagent (Roche Diagnostics K. K.). The results were shown as the mean value ± the standard error. The statistical analysis was performed using Williams' multiple comparison test where the level of statistical significance was less than 5%.

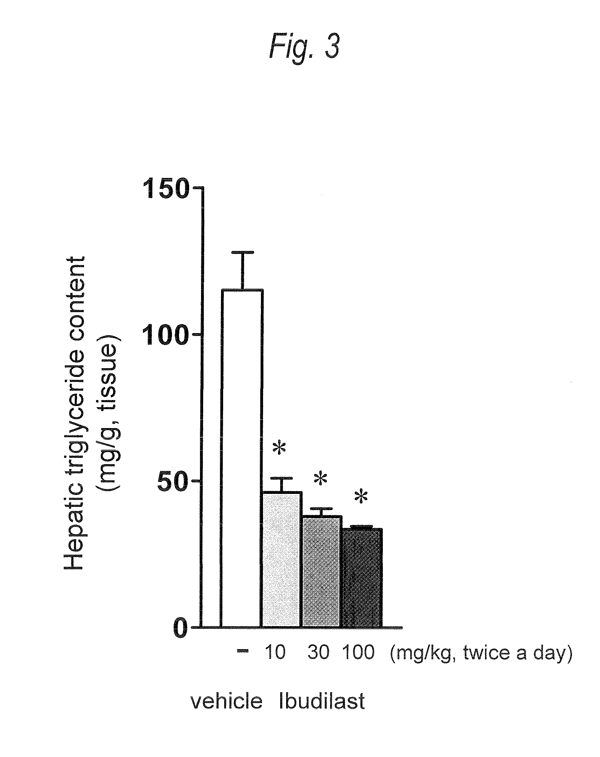

Ibudilast significantly decreased the triglyceride content in liver which is a fatty liver index in obese-related insulin resistance and fatty liver model, ob/ob mice (Fig. 3). Based on the results, it was clearly shown that ibudilast exhibited excellent improvement effects on fatty liver of patients with obesity and insulin resistance.

### Example 3

### Inhibitory effects of ibudilast on hepatic inflammation and hepatic fibrosis

Effects of ibudilast on the hepatic inflammation and hepatic fibrosis were examined using methionine- and choline-deficient (MCD) diet-fed mice which induced hepatic inflammation and hepatic fibrosis involved in human pathological conditions like NASH.

The MCD diet (Oriental Yeast Co., Ltd.) produced in accordance with the previous report by Okumura *et al.* (Okumura, K., et al. 2006, Hepatol. Res., 36: 217-228) was administered to male C57BI/6J mice (CLEA Japan, Inc.) at ten weeks of age for six weeks.

After feeding of MCD diet for six weeks, 2% (v/v) of PEG-60 hydrogenated castor oil (NIKKOL HCO-60, Nikko Chemicals Co., Ltd.) was orally administrated to the vehicle group and 10, 30 or 100 mg/kg of ibudilast which was dissolved to 2% (v/v) of PEG-60 hydrogenated castor oil was orally administered to the ibudilast group twice a day for 14 days.

Thereafter, TNFα, MCP-1, IL-1β, TGFβ, and collal mRNA expressions in isolated liver were measured using the quantitative real-time PCR method. The 18S rRNA mRNA expression was used as an internal standard of the quantitative real-time PCR method and similarly measured.

Liver RNA was extracted using TRIzol reagent (Invitrogen). The DNAase treatment of extracted RNA was performed using RNase-Free DNase Set (Qiagen) end RNeasy mini kit (Qiagen).

The reverse transcription of RNA was performed using High-Capacity cDNA Reverse Transcription kit with RNase inhibitor (Applied Biosystems).

The quantitative real-time PCR was performed using TaqMan Fast Universal PCR Master Mix (Applied Biosystems) and Applied Biosystems 7500 Fast Real-Time PCR System (Applied Biosystems).

TaqMan probes and primers were obtained as assay sets for each target mRNA (TaqMan Gene Expression Assays, Applied Biosystems, Inc.), specifically, TNFα (Mm00443258_ml), MCP-1 (Mm99999056_ml), IL-1β(Mm00434228_ml), TGFβ (Mm01178819_ml) and collal (Mm00801666_gl) of mouse, and 18s rRNA (Hs99999901_sl).

Moreover, each operation relating to the quantitative real-time PCR was carried out in accordance with the manual attached to each reagent, kits and device. The mRNA expression of gene of interest (target) was shown as a relative value in which 18S rRNA was defined as an internal standard. The results were shown as the mean value ± the standard error. The statistical analysis was carried out using Williams' multiple comparison test where the level of statistical significance was less than 5%.

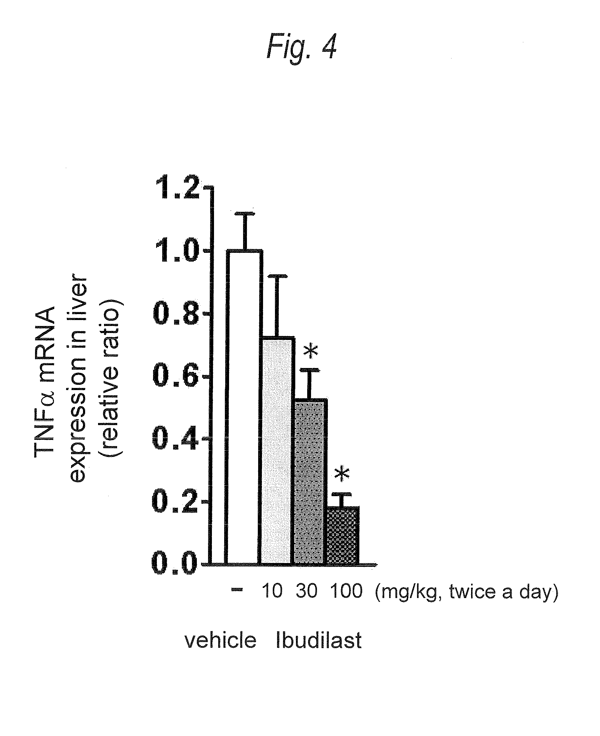

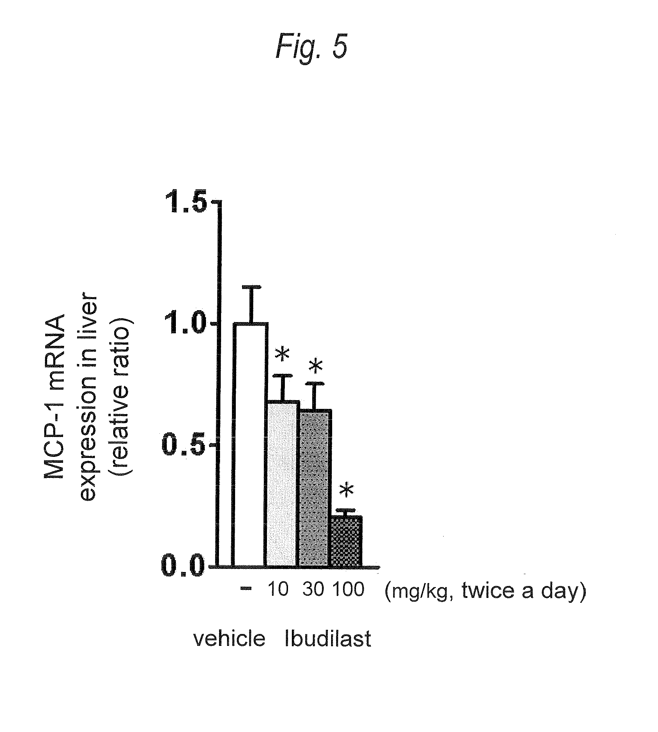

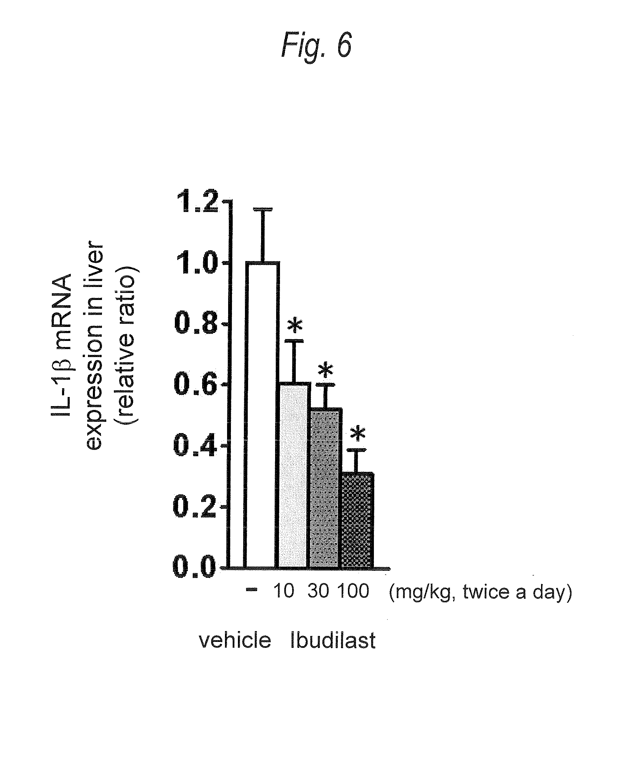

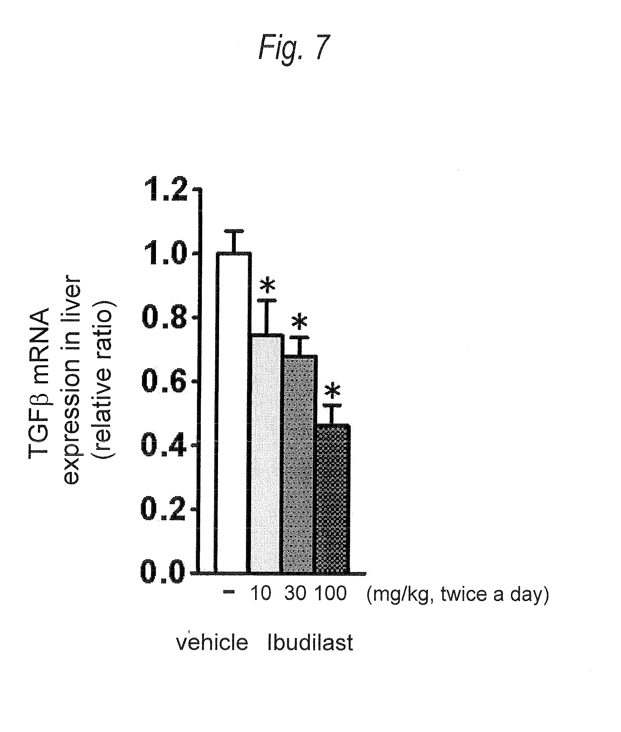

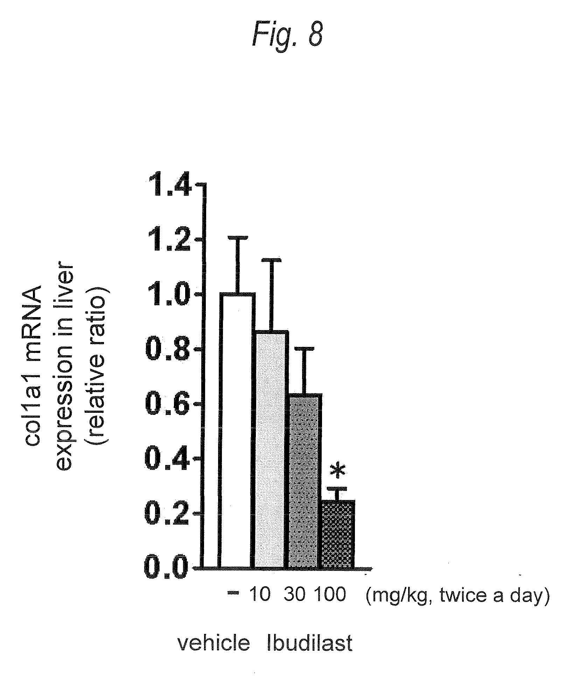

Ibudilast was found to decrease the mRNA expression of TNFα, MCP-1 and IL-1β in liver which were an index of hepatic inflammation, and the mRNA expression of TGFβ and collal in liver which were an index of hepatic fibrosis (Fig. 4 to Fig. 8). Accordingly, it was shown that ibudilast inhibited hepatic inflammation and hepatic fibrosis.

While the invention has been described in detail and with reference to specific embodiments thereof, it will be apparent to one skill in the art that various changes and modifications can be made therein without departing from spirit and scope thereof.
This application is based on Japanese patent application No. 2010-180656 filed on August 12, 2010, the contents of which are incorporated hereinto by reference.

### INDUSTRIAL APPLICABILITY

As a result of the above-mentioned, it was found that ibudilast was improved all of fatty liver, hepatic inflammation and hepatic fibrosis which were main pathological conditions of NASH. Therefore, the agent for prevention or treatment of fatty liver disease of the present invention comprising ibudilast is useful as an agent for prevention or treatment of NAFLD, especially NASH.

## Claims

1. An agent for preventing or treating a fatty liver disease comprising ibudilast as an active ingredient.

2. The agent according to claim 1, wherein the fatty liver disease is nonalcoholic fatty liver disease.

3. The agent according to claim 2, wherein the nonalcoholic fatty liver disease is nonalcoholic steatohepatitis.

4. The agent according to claim 2, wherein the nonalcoholic fatty liver disease is simple fatty liver.
